(19) Europäisches Patentamt
European Patent Office
Office européen des brevets

(11) EP 4 763 833 A1

(12) EUROPÄISCHE PATENTANMELDUNG

(43) Veröffentlichungstag:
**24.06.2026 Patentblatt 2026/26**

(21) Anmeldenummer: **24221208.2**

(22) Anmeldetag: **18.12.2024**

(51) Internationale Patentklassifikation (IPC):
***C07D 233/58** (2006.01)*

(52) Gemeinsame Patentklassifikation (CPC):
**C07D 233/58**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Technikum Laubholz GmbH**
**73033 Göppingen (DE)**

(72) Erfinder: **BISCHOFF, Lukas**
**73434 Aalen (DE)**

(74) Vertreter: **Meissner Bolte Partnerschaft mbB**
**Patentanwälte Rechtsanwälte**
**Postfach 86 06 24**
**81633 München (DE)**

# (54) VERFAHREN ZUR AUFREINIGUNG ODER HERSTELLUNG VON IONISCHEN FLÜSSIGKEITEN UNTER GENERIERUNG EINES ALKOHOLAT-ADDUKT-INTERMEDIATS

(57) Die vorliegende Erfindung betrifft Verfahren zur Aufreinigung oder Herstellung von ionischen Flüssigkeiten des Typs $Im^+Y^-$, wobei von einer verunreinigten ionischen Flüssigkeit $Im^+X^-$ ausgegangen wird. Diese ionische Flüssigkeit wird zunächst mit einem stöchiometrischen Überschuss an Alkali- oder Erdalkalimetallalkoholat versetzt, das in einem organischen Lösungsmittel gelöst ist, das dabei gebildete Alkali- oder Erdalkalimetallsalz wird nachfolgend aus dem Gemisch abgetrennt, das nach der Abtrennung erhaltene Gemisch wird mit einer zur eingesetzten Alkali- oder Erdalkalimetallalkoholat äquimolaren Menge an Säure HY umgesetzt und die dabei gebildeten überschüssigen Salzmengen werden aus der Mischung abgetrennt, um reine ionische Flüssigkeit des Typs $Im^+Y^-$ zu erhalten. Das angegebene Verfahren ermöglicht eine einfache und kostengünstige Herstellung, Aufreinigung und Rückgewinnung von ionischen Flüssigkeiten aus industriellen Prozessen. Die vorliegende Erfindung betrifft weiterhin die Verwendung eines solchen Verfahrens zur Aufreinigung und Rückgewinnung von ionischen Flüssigkeiten, die in Nassspinnverfahren zur Herstellung von Cellulosefilamenten verwendet wurden und Verfahren, die solche Nassspinnverfahren in Kombination mit einer Aufreinigung und Rückgewinnung von ionischen Flüssigkeiten enthalten.

EP 4 763 833 A1

## Beschreibung

Die vorliegende Erfindung betrifft Verfahren zur Aufreinigung oder Herstellung von ionischen Flüssigkeiten des Typs $Im^+Y^-$, wobei von einer verunreinigten ionischen Flüssigkeit $Im^+X^-$ ausgegangen wird. Diese ionische Flüssigkeit wird zunächst mit einem stöchiometrischen Überschuss an Alkali- oder Erdalkalimetallalkoholat versetzt, das in einem organischen Lösungsmittel gelöst ist, das dabei gebildete Alkali- oder Erdalkalimetallsalz wird nachfolgend aus dem Gemisch abgetrennt, das nach der Abtrennung erhaltene Gemisch wird mit einer zur eingesetzten Alkali- oder Erdalkalimetallalkoholat äquimolaren Menge an Säure HY umgesetzt und die dabei gebildeten überschüssigen Salzmengen werden aus der Mischung abgetrennt, um reine ionische Flüssigkeit des Typs $Im^+Y^-$ zu erhalten. Die vorliegende Erfindung betrifft weiterhin die Verwendung eines solchen Verfahrens zur Aufreinigung und Rückgewinnung von ionischen Flüssigkeiten, die in Nassspinnverfahren zur Herstellung von Cellulosefilamenten verwendet wurden und Verfahren, die solche Nassspinnverfahren in Kombination mit einer Aufreinigung und Rückgewinnung von ionischen Flüssigkeiten enthalten.

Stand der Technik

Iminiumsalz-basierte ionische Flüssigkeiten werden bei vielen Prozessen als Lösungsmittel bzw. als Reaktionsmedien eingesetzt (sh. z.B. Ionic Liquids for Clean Technology, K. R. Seddon, J. Chem. Tech. Biotechnol. 1997, 68, 351-356; Room Temperature Molten Salts, N. N. Ene, Roum. Chem. Q. Rev. 1993, 1, 333-351). Bei diesen Anwendungen resultieren nach Abtrennung der gewünschten Produkte ionische Flüssigkeiten, die durch nicht umgesetzte Edukte, zugesetzte Hilfsstoffe oder Reaktionsprodukte verunreinigt sind.

Beim Einsatz ionischer Flüssigkeiten ist deren Reinheit von großer Bedeutung. Verunreinigungen in ionischen Flüssigkeiten können beispielsweise den Verlauf chemischer Reaktionen negativ beeinflussen. So verweisen P. Teisen et al. in Electrochemical Society Proceedings, Vol. 99-41, Seiten 161-168 auf Probleme beim Einsatz von chlorid-haltigen ionischen Flüssigkeiten in der Flüssigphasenhydrierung und bei der Übergangsmetall-katalysierten Suzuki-Reaktion, die auf Verunreinigungen zurückführen sind. Daher sind bei der Herstellung ionischer Flüssigkeiten hohe Anforderungen an die Reinheit der gewünschten Flüssigkeit zu stellen.

Eine ausreichende Reinheit kann zwar in frisch hergestellten oder kommerziell erhältlichen ionischen Flüssigkeiten sichergestellt werden, ionische Flüssigkeiten, die nur einmal verwendet werden, stellen aber einen signifikanten Kostenfaktor dar, der die Umstellung von industriellen Prozessen auf die Verwendung von ionischen Flüssigkeiten unattraktiv macht. Ein Recycling von verunreinigten ionischen Flüssigkeiten bzw. die Rückgewinnung von möglichst reinen ionischen Flüssigkeiten aus verunreinigten Chargen stellt wegen der besonderen Eigenschaften der Flüssigkeiten ein erhebliches Problem dar. So besitzen die Verbindungen zumeist Siedepunkte bzw. Sublimationspunkte, die deutlich über 300 °C liegen. Nur ionische Flüssigkeiten, die spezielle Anionen wie z.B. $N(SO2F)_2$ enthalten, sind bei diesen Temperaturen einigermaßen stabil, so dass sie oft im Kleinmaßstab destillierbar sind. Auch dies ist jedoch mit einem erheblichen Energiebedarf verbunden. Vor diesem Hintergrund besteht ein Bedarf für ein allgemein anwendbares Reinigungs- oder Recyclingverfahren, das auch bei tieferen Temperaturen (<200 °C) durchführbar ist.

Die Aufreinigung und das Recycling von verunreinigten ionischen Flüssigkeiten ist insbesondere für ionische Flüssigkeiten von Interesse, die bei Verfahren zur Verspinnung von Cellulose in Nassspinnanlagen eingesetzt werden. Bei einem solchen Prozess wird die Cellulose bei erhöhter Temperatur in der ionischen Flüssigkeit gelöst. Diese Lösung wird durch Düsen in ein Fällbad gepresst, das aus wässrigen bzw. alkoholischen Lösungen von Alkalisalzen, schwachen Säuren, Tensiden usw. bestehen kann. Nach Ende des Spinnvorgangs wird das Fällbad vom Lösungsmittel (Wasser, Alkohol) befreit und die zurückbleibende IL (= ionische Flüssigkeit) erneut verwendet. Die im Fällbad vorhandenen Hilfsstoffe, wie z.B. Alkalimetallchloride, -sulfate, -acetate sowie Wasser- oder Alkoholreste usw. verbleiben in der ionischen Flüssigkeit. Zu weiteren Verunreinigungen führt auch die Tatsache, dass beim Auflösen in der heißen ionischen Flüssigkeit ein Teil der Cellulose depolymerisiert, so dass die Celluloselösung oligomere Glucosen enthält, die beim Spinnvorgang nicht ausgefällt werden und nach dem Eindampfen des Fällbads in der ionischen Flüssigkeit zurückbleiben. Die Menge an Verunreinigungen steigt somit bei der mehrfachen Verwendung in der wiedergewonnenen ionischen Flüssigkeit stetig an.

In solchen Verfahren werden heute insbesondere Imidazoliumcarboxylate als ionische Flüssigkeiten eingesetzt, was infolge der Möglichkeit eines Verzichts auf konventionell verwendete deutlich umweltschädlichere Lösemittel (z.B. $NaOH/CS_2$/Wasser oder N-Methylmorpholin-oxid) mit erheblichen Vorteilen verbunden ist. Derartige Nassspinnverfahren sind wegen der hohen Gestehungskosten der ionischen Flüssigkeiten allerdings nur dann wirtschaftlich betreibbar, wenn die ionischen Flüssigkeiten mehrmals verwendet werden können und nach der mehrmaligen Verwendung eine Möglichkeit für eine kostengünstige Aufarbeitung und Reinigung der Flüssigkeiten besteht. Dies gilt insbesondere vor dem Hintergrund, dass bei einer industriellen Herstellung von regenerierter Cellulose ionische Flüssigkeiten im Tonnenmaßstab benötigt werden.

Die WO 2005/070896 A1 beschreibt ein Verfahren zur Herstellung von ionischen Flüssigkeiten, bei dem von

einer ionischen Flüssigkeit des Typs $Im^+Hal^-$ (Hal = Halogen) ausgegangen wird und die ionische Flüssigkeit zunächst mit einer äquimolaren Menge an Alkalimetallalkoholat, Carboxylat oder Bariumhydroxid umgesetzt wird, um ein entsprechendes Intermediat ImOR, ImOCOR oder ImOH zu bilden. Dieses Intermediat wird von den in der Umsetzung gebildeten Salzen, die in der Reaktionsmischung unlöslich sind, abgetrennt und mit einer äquimolaren Säuremenge neutralisiert. In den Ausführungsbeispielen der WO 2005/070896 A1 wird dazu eine Probe des gebildeten Zwischenprodukts titriert um die erforderliche Säuremenge zu bestimmen.

Das in der WO 2005/070896 A1 beschriebene Verfahren weist den Nachteil auf, dass es vergleichsweise aufwendig ist und nicht ersichtlich ist, wie das Verfahren für die Aufreinigung von verunreinigten ionischen Flüssigkeiten oder zur Herstellung von ionischen Flüssigkeiten aus verunreinigten Ausgangsmaterialien genutzt werden könnte.

Die WO 2024/153790 A1 beschreibt Verfahren zur Herstellung von ionischen Flüssigkeiten, bei dem in ähnlicher Weise eine ionische Flüssigkeit des Typs $Im^+X^-$ (wobei $X^-$ verschiedene Anionen in Form von Halogeniden, Carboxylaten, Phosphaten, etc. bezeichnet) als Ausgangsmaterial zunächst mit einem Alkalimetallalkoholat umgesetzt, das dabei gebildete Salz von dem ebenfalls gebildetem ImOR Intermediat abgetrennt und das Intermediat ImOR dann mit einer Säure umgesetzt wird. Durch die verwendetet Säure wird dabei das Endprodukt $Im^+Y^-$ bestimmt, das bei $X^- = Y^-$ mit der als Ausgangsprodukt verwendeten ionischen Flüssigkeit identisch sein kann, oder bei $X^- \neq Y^-$ eine ionische Flüssigkeit mit einem anderen Anion ist. In den in WO 2024/153790 A1 beschriebenen Beispielen wird die als Ausgangsprodukt verwendete ionische Flüssigkeit ebenfalls mit einer äquimolaren Menge an Alkalimetallalkoholat umgesetzt.

Nach den beschriebenen Vorgehensweisen besteht der erste Schritt für eine Umsetzung in der Bestimmung des tatsächlichen Gehalts der ionischen Flüssigkeit (oder des aus dieser gebildeten ImOH Intermediats). Während sich diese bei den verwendeten reinen Ausgangsprodukten vergleichsweise einfach bewerkstelligen lässt, gestaltet sie sich bei verunreinigten ionischen Flüssigkeiten, die bei Prozessen anfallen, deutlich schwieriger. Hier können zwar analytische Methoden wie $^1$H- NMR oder HPLC-MS verwendet werden, die aber vergleichsweise teure Apparaturen erfordern und nicht in jedem industriellen Betrieb vorhanden sind. Auch ist in den Dokumenten nicht beschrieben, wie Verunreinigungen über die angegebenen Verfahren zur Herstellung von ionischen Flüssigkeiten abgetrennt werden können.

Vor diesem Hintergrund besteht ein Bedarf für ein kostengünstiges und einfach zu implementierendes Verfahren zur Herstellung von ionischen Flüssigkeiten des Typs $Im^+Y^-$ aus verunreinigten Mischungen einer als Ausgangsprodukt verwendeten ionischen Flüssigkeit $Im^+X^-$, bei dem aufwändige Gehaltsbestimmungen der ionischen Flüssigkeit und von gegebenenfalls zwischenzeitlich gebildeten Intermediaten nicht erforderlich sind. Es besteht weiterhin ein Bedarf für ein Verfahren, bei dem die in den verunreinigten Ausgangsprodukten enthaltenen Verunreinigungen im Verlauf des Verfahrens möglichst weitgehen abgetrennt werden können. Die vorliegende Erfindung befasst sich mit diesem Bedarf.

Detaillierte Beschreibung der Erfindung

In den der vorliegenden Erfindung zugrundeliegenden Untersuchungen wurde gefunden, dass sich eine Bestimmung des Gehalts einer ionischen Flüssigkeit in einer Charge, die weitere Verunreinigungen enthält, vermeiden lässt, indem die verunreinigte ionische Flüssigkeit zunächst absichtlich mit einem Überschuss an Alkali- oder Erdalkalimetallalkoholat umgesetzt wird. Dies hat zwar zur Folge, dass zunächst ein Zwischenprodukt gebildet wird, dass noch überschüssige Anteile an Alkali- oder Erdalkalimetallalkoholat enthält, dies bleibt aber zusammen mit dem aus der ionischen Flüssigkeit gebildeten Alkoholat-Addukt des Kations der ionischen Flüssigkeit in Lösung, während aus dem Kation des Alkali- oder Erdalkalimetallalkoholats und dem Anion der ionischen Flüssigkeit gebildetes Salz aus der Lösung ausfällt und abgetrennt werden kann. Auch stark polare Verunreinigungen können auf diese Weise als Feststoff aus der Mischung abgetrennt werden.

Das Gemisch aus Alkoholat-Addukt des Kations der ionischen Flüssigkeit in Lösung und überschüssigem Alkali- oder Erdalkalimetallalkoholat wird anschließend mit einer in Bezug auf die eingesetzte Menge des Alkali- oder Erdalkalimetallalkoholats äquimolaren Menge einer Protonensäure umgesetzt, wobei sich aus dem Alkoholat-Addukt und dem überschüssigen Alkali- oder Erdalkalimetallalkoholat der entsprechende Alkohol und Alkali- oder Erdalkalimetallsalz der zugesetzten Säure bildet. Der Alkohol kann im Unterdruck oder destillativ aus dem gebildeten Produkt entfernt werden, während das Alkali- oder Erdalkalimetallsalz infolge seiner im Vergleich zur ionischen Flüssigkeit deutlich geringere Löslichkeit von dieser abgetrennt werden kann. Dies kann gegebenenfalls durch die Zugabe eines im Vergleich zur ionischen Flüssigkeit unpolareren organischen Lösungsmittels unterstützt werden, in dem sich die ionische Flüssigkeit löst. Relevante polare Verunreinigungen können dabei auf der Stufe der Abtrennung der Salze mit diesen entfernt werden.

Gemäß einem ersten Aspekt betrifft die vorliegende Erfindung demzufolge ein Verfahren zur Aufreinigung oder Herstellung von ionischen Flüssigkeiten des Typs $Im^+Y^-$ mit den folgenden Schritten:

i) Umsetzen einer Mischung, die eine ionische Flüssigkeit $Im^+X^-$ und eine oder mehrere Verunreinigungen enthält, mit einem stöchiometrischen Überschuss an Alkali- oder Erdalkalimetallalkoholat, das in einem organischen Lösungsmittel gelöst ist,

ii) Abtrennen des bei der Umsetzung erzeugten Salzes aus Alkali- oder Erdalkalimetallkation und $X^-$ aus der

Reaktionsmischung,

iii) Umsetzen des resultierenden Produkts mit einer zur eingesetzten Alkali- oder Erdalkalimetallalkoholat äquimolaren Menge an Säure HY, und

iv) Abtrennen von hierbei generiertem Alkohol und überschüssigem Salz aus Alkalimetall und $Y^-$ aus der Reaktionsmischung.

Die Angabe "stöchiometrischer Überschuss" bezeichnet den Umstand, dass die molare Menge der ionischen Flüssigkeit in der in i) verwendenden Mischung geringer ist als die molare Menge des zugesetzten Alkali- oder Erdalkalimetallalkoholats. In der Regel ist es ausreichend, wenn dieser Überschuss bei etwa 1% liegt um sicherzustellen, dass die gesamte Menge an in der Mischung enthaltener ionischer Flüssigkeit in ein Addukt aus Alkoholat und Kation der ionischen Flüssigkeit umgewandelt werden kann. Der "stöchiometrische Überschuss" ist nach oben nicht begrenzt, da ein höherer Überschuss aber eine ungünstigerer Verfahrensökonomie zur Folge hat, wird der Fachmann bestrebt sein, den zugesetzten Überschuss auf das für das Erreichen des erfindungsgemäßen Zweckes Notwendige zu begrenzen.

Ein besonders günstiges Verfahren zu Bestimmung des stöchiometrischen Überschusses besteht darin, anzunehmen, dass es sich bei der verunreinigten ionischen Flüssigkeit um eine reine ionische Flüssigkeit handelt, und deren angenommenen Molmenge durch Teilen der umzusetzenden Gewichtsmenge an verunreinigter ionischer Flüssigkeit durch das Molekulargewicht der ionischen Flüssigkeit zu ermitteln. Da die wenigsten Verunreinigungen, die in ionischen Flüssigkeiten anfallen, selbst mit Alkoholaten reagieren, führt ein solcher Ansatz dazu, dass die Menge an für die Umsetzung benötigtem Alkali- oder Erdalkalimetallalkoholat überschätzt wird, und damit automatisch eine Menge berechnet wird, die die für eine vollständige Umsetzung der Kationen der ionischen Flüssigkeit zu Alkoholat-Addukten erforderliche Alkoholatmenge übersteigt (wenn die Molmenge an Hand der Annahme eines Gehalts der ionischen Flüssigkeit von 100% berechnet wird, die ionische Flüssigkeit tatsächlich aber nur 95% der Menge des eingesetzten Produkts ausmacht, wird der Anteil an erforderlichem Alkali- oder Erdalkalimetallalkoholat um 5% überschätzt und entsprechende eine um etwa 5,3 % größere Menge als Alkoholat zugesetzt, als dies erforderlich wäre). Eine solche Vorgehensweise hat darüber hinaus den Vorteil, dass sich ein Ausschluss von Wasser in einem allein auf Abwiegen beruhenden Verfahren relativ leicht realisieren lässt. Dies stellt insbesondere für stark hygroskopische Iminium- und Oniumsalzen einen erheblichen Vorteil dar, da auf die Einhaltung oder eine Handhabung im einer wasserfreien Schutzgasatmosphäre verzichtet werden kann.

Im Rahmen des angegebenen Verfahrens handelt es sich in einer bevorzugten Ausführungsform bei dem Kation der ionischen Flüssigkeit um ein Imidazoliumkation, so dass es sich bei den ionischen Flüssigkeiten $Im^+X^-$ und $Im^+Y^-$ jeweils um Imidazoliumsalze handelt. Die ionische Flüssigkeit kann jedoch auch ein Kation aufweisen, dass nicht auf einem Imidazolring beruht, wie z.B. einem N,O-bisalyklierten Pyrrolidon, $R^1$-$C(NR^2{}_2)_2{}^+$, $R^1$-$C(NR^2)(SR_2)^+$ oder einem Guanidiniumkation ($C(NR^2{}_2)_3{}^+$), wobei $R^2$ in den gezeigten Strukturen gleich oder verschieden sein kann, und einen Kohlenwasserstoffrest, bevorzugt in Form eines Alkylrestes, bezeichnet und wobei $R^1$ einen Kohlenwasserstoffrest, bevorzugt in Form eines Alkylrestes, bezeichnet. Weitere mögliche Iminiumsalze, die im Rahmen des hier beschriebenen Verfahrens behandelt werden können, umfassen die nachfolgend dargestellten 1,3-Dialkyl-1,3,4-triazoliumsalze (**1**), 2,3,5-Trialkyl-1,3-oxazoliumsalze (**2**), 2,3,5-Trialkyl-1,3,4-oxdiazoliumsalze (**3**), 2,3,5-Trialkyl-1,3-thiazoliumsalze (**4**), 2,3,4-Trialkyl-1,3,4-thiadiazoliumsalze (**5**), 1,2-Dialkylpyrimidiniumsalze (**6**) und 1,2,3-Trialkyl-3,4,5,6-tetrahydro-pyrimidiniumsalze (**7**):

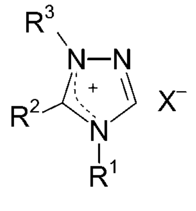

**1**

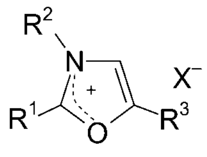

**2**

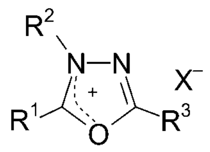

**3**

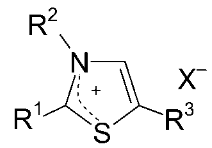

**4**

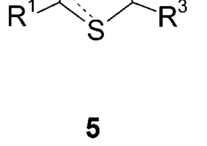

**5**

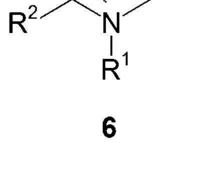

**6**

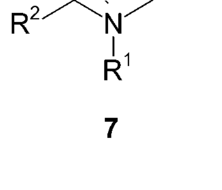

**7**

Alternativ sind auch ionischen Flüssigkeiten auf der Basis von Kationen verwendbar, wie sie in der WO2005/070896 auf den Seiten 4 bis 6 beschrieben sind.

Wenn die im erfindungsgemäßen Verfahren verwendete ionische Flüssigkeit ein Imidazoliumsalz ist, ist es bevorzugt, wenn das in dem Salz enthaltene Imidazoliumkation an den Stickstoffatomen (1'- und 3'-Position) substituiert ist, und vorzugsweise an der 4'- und 5'-Position unsubstituiert ist. Das Kohlenstoffatom an der 2'-Position kann unsubstituiert sein (d.h. ein zusätzlich gebundenes Wasserstoffatom aufweisen, $R^2$ = H) oder substituiert sein, beispielsweise mit einer Alkylgruppe, die ein oder mehrere Substituenten ausgewählt aus Alkyl, insbesondere C1-C6-Alkyl, Alkoxy, insbesondere C1-C6-Alkoxy, Aryl, insbesondere Phenyl enthalten kann. Alternativ kann das Kohlenstoffatom an der 2'-Position mit einem Arylrest (z.B. in Form von Phenyl) oder mit $NR_2$ substituiert sein, wobei R ein Alkyl- oder Arylrest sein kann, und die beiden R gleich oder verschieden sein können. Bevorzugt ist R ein C1-C4 Alkylrest und ganz besonders bevorzugt ist R = Methyl. Die Positionen der Reste ergeben sich aus der nachfolgenden Abbildung eines Imidazoliumsalzes **8:**

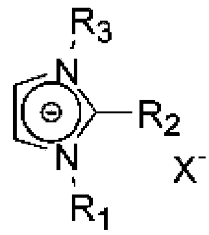

**8**

In dieser bezeichnet die 2'-Position die Position im Immidazolring mit dem Substituenten $R_2$, die 1'-Position die Position im Immidazolring mit dem Substituenten $R_1$ und die 3'-Position die Position im Immidazolring mit dem Substituenten $R_3$. Die Positionen 4' und 5' sind unsubstituiert (bzw. weisen einen Wasserstoffsubstituenten auf)

In einer besonders bevorzugten Ausführungsform ist das Imidazoliumkation des Imidazoliumsalzes in der 1'- sowie 3'-Position (entsprechend den Resten $R^1$ und $R^3$ im Reaktionsschema oben) oder in der 1'-, 2'- sowie 3'-Position (entsprechend den Resten $R^1$, $R^2$ und $R^3$ im Reaktionsschema oben) mit (C1-C6)-Alkylgruppen substituiert ist, wobei eine 2'-Position neben Alkyl auch mit Aryl oder Alkylaryl substituiert sein kann, und die Alkylgruppe einen Alkyloxy- oder Aryloxysubstituenten aufweisen kann. $R^1$ und $R^3$ können gleich oder verschieden sein. In einer noch weiter bevorzugten Ausführungsform ist das Imidazoliumkation des Imidazoliumsalzes ausgewählt aus der Gruppe bestehend aus 1-Ethyl-3-methylimidazolium-, 1,3-Dimethylimidazolium-, 1-Butyl-3-methylimidazolium-Kation-, 1-Butyl-3-ethylimidazolium-, 1,3-Diethylimidazolium-, 1-Ethyl-3-isopropylimidazolium-, 1-Allyl-3-methylimidazolium-, 1-Allyl-3-ethylimidazolium-, 1-Allyl-3-isopropylimidazolium-, 1-Allyl-3-butylimidazolium-Kationen.

Das Anion $X^-$ der ionischen Flüssigkeit, die in der Ausgangsmischung in Schritt i) des erfindungsgemäßen Verfahrens verwendet wird, ist bevorzugt ausgewählt aus der Gruppe umfassend $F^-$, $Cl^-$, $Br^-$, $ClO_3^-$, $ClO_4^-$, $CN^-$, $SCN^-$, $NO_2^-$, $NO_3^-$, $HCO_3^-$, $CO_3^{2-}$ , $HSO_4^-$, $SO_4^{2-}$, $S_2O_3^{2-}$, $S_2O_4^{2-}$, $S_2O_5^{2-}$, $S_2O_6^{2-}$, $HPO_2^-$, $H_2PO_4^-$, $HPO_4^{-2-}$, $RN-CS_2^-$, $BF_4^-$, $SbCl_6^-$, $AlCl_4^-$, $FeCl_4^-$,

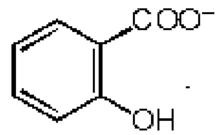 ,

$C_nH_{2n+1}CO_2^-$, $C_nH_{2n-1}CO_2^-$, $C_nH_{2n-3}CO_2^-$, $^-O_2C-(CH_2)_n-CO_2H$, $^-O_2C-(CH_2)_n-CO_2^-$ mit n = 1 bis 25, $R-SO_3^-$, $CF_3SO_3^-$, $C_6H_5SO_3^-$, $C_nH_{2n-1}CO_2^-$,

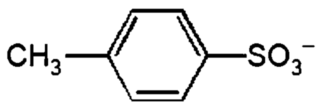 ,

und **$Ar-CO_2^-$ (Ar** = $C_6H_5$,

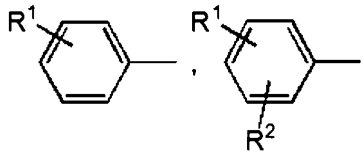

).

Wenn es sich bei dem erfindungsgemäßen Verfahren um ein Verfahren zur Herstellung von ionischen Flüssigkeiten handelt (was in dem hier beschrieben Kontext bedingt, dass die ionische Flüssigkeit, die im Verfahren als Ausgangsprodukt eingesetzt wird ≠ der ionischen Flüssigkeit ist, die als Endprodukt des Verfahrens erhalten wird, bzw. $X^- \neq Y^-$), ist das Anion der ionischen Flüssigkeit bevorzugt ein Halogenid und insbesondere Chlorid. Ionische Flüssigkeiten auf Basis von Chloriden stellen die am besten kommerziell verfügbaren ionischen Flüssigkeiten dar, so dass solche ionischen Flüssigkeiten (in verunreinigter Form) ein leicht verfügbares Ausgangsprodukt darstellen.

Im Sinne einer möglichst weitgehenden Abtrennung von Alkali- oder Erdalkalimetallsalzen in Schritt ii) ist es weiterhin bevorzugt, wenn es sich bei dem Anion um ein anorganisches Anion handelt, zu denen die Halogenide $F^-$, $Cl^-$, und $Br^-$, sowie $ClO_3^-$, $ClO_4^-$, $CN^-$, $SCN^-$, $NO_2^-$, $NO_3^-$, $HCO_3^-$, $CO_3^{2-}$, $HSO_4^-$, $SO_4^{2-}$, $S_2O_3^{2-}$, $S_2O_4^{2-}$, $S_2O_5^{2-}$, $S_2O_6^{2-}$, $HPO_2^-$, $H_2PO_4^-$, $HPO_4^{-2-}$, $RN\text{-}CS_2^-$, $BF_4^-$, $SbCl_6^-$, $AlCl_4^-$, $FeCl_4^-$, $CF_3SO_3^-$ und

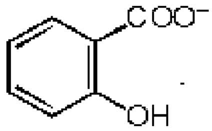

zu zählen sind.

Wenn es sich bei dem Anion um ein organisches Anion handelt, dass eine höhere Liphophilie aufweist als anorganische Anionen, kann es zweckmäßig sein, die Abscheidung von entprechenden Alkali- oder Erdalkalimetallsalzen durch die Zugabe eines Lösungsmittels, dass unpolarer ist als das in Schritt i) verwendetet organische Lösungsmittel, zu unterstützen (wenn beispielsweise in Schritt i) ein Alkohol verwendet wird, kann in Schritt ii) ein unpolarerer Ester zugegeben werden).

Organische Anionen im Kontext der hier angegebenen Erfindung sind beispielsweise $C_nH_{2n+1}CO_2^-$, $C_nH_{2n-1}CO_2^-$, $C_nH_{2n-3}CO_2^-$, $^-O_2C\text{-}(CH_2)_n\text{-}CO_2H$, $^-O_2C\text{-}(CH_2)_n\text{-}CO_2^-$ mit n = 1 bis 25, $R\text{-}SO_3^-$, $C_6H_5SO_3^-$, $C_nH_{2n-1}CO_2^-$,

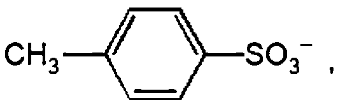

und **Ar-$CO_2^-$ (Ar** = $C_6H_5$,

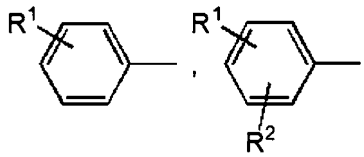

).

Hinsichtlich der Verunreinigungen, die in der als Ausgangsprodukt verwendeten Mischung enthalten sein können, unterliegt die vorliegende Erfindung keinen relevanten Beschränkungen. Bevorzugt ist es, wenn die in Schritt i) des Verfahrens als Ausgangsprodukt eingesetzte Mischung als Verunreinigung eine oder mehrere Stoffe enthält, die aus der Gruppe bestehend aus Alkali- oder Erdalkalimetallsalzen, Mono- und/oder Oligosacchariden, insbesondere in Form von oligomeren Glucosen oder oligomeren Xylosen, Proteinen und Schwebstoffe ausgewählt sind. Die enthaltenen Verunreinigungen sind in der Regel in der ionischen Flüssigkeit zumindest teilweise löslich, aber andererseits bevorzugt so polar, dass sie sich nicht in einem homogenen Gemisch (bzw. einer Lösung) der ionischen Flüssigkeit mit einem weniger polaren Lösungsmittel lösen. Es ist auch möglich, dass die Mischung Verunreinigungen enthält, die weniger polar sind als die ionische Flüssigkeit, die Entfernung solcher Verunreinigungen kann jedoch in der Regel durch Extraktion der ionischen Flüssigkeit mit einem stark unpolaren Lösungsmittel realisiert werden, in dem sich die ionische Flüssigkeit selbst nicht löst. Eine solche Extraktion kann zweckmäßig durchgeführt werden, bevor die Ionische Flüssigkeit einer Behandlung gemäß dem hier angegebenen Verfahren unterzogen wird, um auf diese Weise die Menge des im Verfahren

eingesetzten Alkali- oder Erdalkalimetallalkoholats und der Säure HY minimieren zu können.

Hinsichtlich der Menge der in der Mischung enthaltenen Verunreinigung unterliegt das Verfahren der vorliegenden Erfindung ebenfalls keinen relevanten Beschränkungen. In dem meisten Fällen liegt deren Anteil in der verwendeten Mischung im Bereich von 2 bis 40 Gew.-% und insbesondere 5 bis 30 Gew.-%.

Das im Schritt i) des angegebenen Verfahrens verwendete Lösungsmittel dient dem Zweck einer Verschiebung der Polarität der Mischung in einen weniger polaren Bereich, so dass eine Separation von festem Alkali- oder Erdalkalimetallsalz auf der Mischung erleichtert wird. Zu diesem Zweck geeignete Lösungsmittel, weisen eine Eigenpolarität auf, die sicherstellt, dass zumindest im Gemisch mit der ionischen Flüssigkeit eine ausreichende Löslichkeit von überschüssigem Alkali- oder Erdalkalimetallalkoholat gewährleistet ist, und die mit der ionischen Flüssigkeit homogen (d.h. unter Ausbildung einer Mischphase) mischbar sind. Beispielhaft als verwendbare Lösungsmittel können dabei insbesondere die Folgenden genannt werden: Methanol, Ethanol, Methylacetate, Ethylacetat und Acetonitril. Von diesen gelten Lösungsmittel in Form von Estern als weniger bevorzugt, da diese in Anwesenheit von Restwasser mit den Alkali- oder Erdalkalimetallalkoholaten zu Carboxylaten reagieren können. Ganz besonders bevorzugt als organische Lösungsmittel sind Alkohole, insbesondere in Form von Methanol und Ethanol, die eine gute Löslichkeit für Alkali- oder Erdalkalimetallalkoholate ausweisen und die kostengünstig und in großen Mengen verfügbar sind.

Die Menge des organischen Lösungsmittels, das in Schritt i) des erfindungsgemäßen Verfahrens verwendet wird, ist durch den Fachmann zweckmäßig so einzustellen, dass einerseits nicht zu große Mengen eingesetzt werden (was das Verfahren unwirtschaftlich machen würde) und andererseits die gewünschte Verschiebung der Polarität der Mischung erreicht wird, um eine Abscheidung von Verunreinigungen und Salzen aus der Mischung zu erleichtern. Hierbei ist es auch möglich, der Reaktionsmischung Anteile von unpolareren Lösungsmitteln (z.B. in Form von Ethern) zuzugeben, solange gewährleistet ist, dass überschüssiges Alkali- oder Erdalkalimetallalkoholat in der Mischung gelöst bleibt.

Als ein geeignetes Mengenverhältnis von organischem Lösungsmittel zu ionischer Flüssigkeit inklusive darin enthaltener Verunreinigungen in Schritt i) kann ein Bereich von 5:1 bis 1:1, und bevorzugt 4:1 bis 1,5:1 eingestellt werden. Bei diesen Verhältnissen handelt es sich um Verhältnisse nach Gewicht.

Das Alkoholat, das gemäß dem erfindungsgemäßen Verfahren in Schritt i) zu der Mischung aus ionischer Flüssigkeit $Im^+X^-$ und einer oder mehrerer Verunreinigungen zugegeben wird, ist zweckmäßig ein C1-C4 Alkoholat, wobei Methanolat und Ethanolat ganz besonders bevorzugt sind. Als Alkali- oder Erdalkalimetallkation des Alkoholats sind Natrium oder Kalium bevorzugt. Dabei ist Natrium aus Kosten- und Verfügbarkeitsgründen meist bevorzugt. Verwendbare Alkoholate auf der Basis von Erdalkalikationen sind insbesondere Mg- oder Ca-Alkoholate.

Das Abtrennen des Salzes, das sich aus dem Kation des Alkoholats und dem Anion der ionischen Flüssigkeit gebildet hat, kann in Schritt ii) mit Hilfe jedes für die Abtrennung von festen oder dichteren Rückständen geeigneten Verfahrens durchgeführt werden. In einem Beispiel erfolgt das Abtrennen des Salzes durch Filtrieren, bevorzugt bei Umgebungstemperatur (20 bis 28°C) oder einer geringeren Temperatur oberhalb der Abscheidungstemperatur des Addukts des Kations der ionischen Flüssigkeit und dem Alkoholat und oberhalb der Abscheidungstemperatur von Alkali- oder Erdalkalimetallalkoholat. Dabei bezeichnet die "Abscheidungstemperatur" die Temperatur, in der die bezeichneten Substanzen nicht mehr gelöst sind, d.h. z.B. in Folge von Kristallisation oder Phasentrennung. In einer anderen Ausführungsform erfolgt das Abtrennen durch Abdekantieren der Lösung des Addukts des Kations der ionischen Flüssigkeit und dem Alkoholat, gegebenenfalls nach Zentrifugieren, um nicht gelöste Bestandteile an Boden eines Reaktionsgefäßes zu konzentrieren.

Sowohl für eine Abtrennung durch Filtrieren als auch für ein Abtrennen durch Abdekantieren und/oder Zentrifugieren und Entnahme der überstehenden Lösung des Addukts des Kations der ionischen Flüssigkeit und dem Alkoholat ist es zur Optimierung der Ausbeute der am Ende des Verfahrens erhaltenen ionischen Flüssigkeit zweckmäßig, ausgefallene Salzprodukte mit Lösungsmittel zu waschen, um darin noch enthaltenen Reste des Addukts und des Alkali- oder Erdalkalimetallalkoholats zu extrahieren. Hierzu können die Salzprodukte beispielsweise mit der gleichen Menge oder eine größere Menge an Lösungsmittel gewaschen werden, die in der Mischung mit der ionischen Flüssigkeit in Schritt i) verwendet wurde, wobei auch ein mehrfaches Waschen möglich ist. Zweckmäßig wird für diese Behandlung das gleiche Lösungsmittel verwendet, das in Schritt i) verwendet wurde, da auf diese Weise eine nachfolgende aufwändigere Trennung von Lösungsmittelgemischen vermieden werden kann.

Die Säure, die in Schritt iii) des erfindungsgemäßen Verfahrens zugegeben wird, ist zweckmäßig eine Protonensäure, die von einem Anion ausgewählt aus der folgenden Gruppe abgeleitet ist:

$F^-$, $Cl^-$, $Br^-$, $ClO_3^-$, $ClO_4^-$, $CN^-$, $SCN^-$, $NO_2^-$, $NO_3^-$, $HCO_3^-$, $CO_3^{2-}$, $HSO_4^-$, $SO_4^{2-}$, $S_2O_3^{2-}$, $S_2O_4^{2-}$, $S_2O_5^{2-}$, $S_2O_6^{2-}$, $HPO_2^-$, $H_2PO_4^-$, $HPO_4^{-2-}$, $RN\text{-}CS_2^-$, $BF_4^-$, $SbCl_6^-$, $AlCl_4^-$, $FeCl_4^-$,

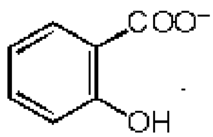
,

$C_nH_{2n+1}CO_2^-$, $C_nH_{2n-1}CO_2^-$, $C_nH_{2n-3}CO_2^-$, $^-O_2C-(CH_2)_n-CO_2H$, $^-O_2C-(CH_2)_n-CO_2^-$ mit n = 1 bis 25, $R-SO_3^-$, $CF_3SO_3^-$, $C_6H_5SO_3^-$, $C_nH_{2n-1}CO_2^-$,

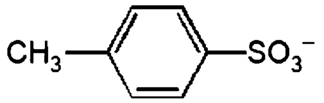 ,

und **Ar-$CO_2^-$ (Ar** = $C_6H_5$,

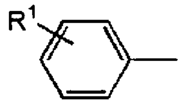 , 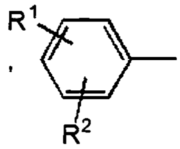

).

Im erfindungsgemäßen Herstellungsverfahren handelt es sich bei der Säure in einer bevorzugten Ausführungsform um eine organische Säure, bevorzugt um eine Carbonsäure, und weiter bevorzugt um eine ungesättigte, oder eine ein- oder mehrfach ungesättigte Carbonsäure. Die Angabe "Carbonsäure" umfasst hier Carbonsäuren mit einer (z.B. $C_nH_{2n+1}CO_2H$) oder mehreren, insbesondere zwei Carbonsäurefunktionen (z.B. $HO_2C-(CH_2)_n-CO_2H$).

Ganz besonders bevorzugt ist es im Rahmen des erfindungsgemäßen Verfahrens, wenn es sich bei der Säure um eine C4 bis C20 Carbonsäure (d.h. eine ungesättigte, oder eine ein oder mehrfach ungesättigte Carbonsäure) handelt, wobei C6 bis C18 Carbonsäuren als noch weiter bevorzugt angegeben werden können. Derartige Carbonsäuren weisen den Vorteil auf, dass ihre Salze in vielen organischen Lösungsmitteln wie Alkoholen, Ethern, Carbonsäureestern, Ketonen und Nitrilen praktisch unlöslich sind, so dass diese Salze aus entsprechenden Lösungen der ionischen Flüssigkeiten quantitativ ausfallen und nachfolgend abgetrennt werden können.

Wie vorstehend erwähnt, wird die Saure HY in einer Menge eingesetzt, die zur eingesetzten Menge and Alkali- oder Erdalkalimetallalkohol äquimolar ist (d.h. wenn 1,00 mol Alkali- oder 0,50 mol Erdalkalimetallalkoholat in Schritt i) eingesetzt wird, wird 1,00 mol der Säure HY in Schritt iii) eingesetzt).

Die in Schritt iii) zugesetzte Säure HY kann bei einer geeigneten Temperatur zugegeben werden, die abhängig von der Reaktionsfreudigkeit des Addukts des Kations der ionischen Flüssigkeit und dem Alkoholat und der Stabilität gegen unerwünschte Zersetzung eingestellt werden kann. In einer Ausführungsform wird die Säure bei Umgebungstemperatur (20 bis 28°C) zugegeben. In einer anderen Ausführungsform wird die Säure bei verminderter Temperatur, z.B. bei -30°C bis 15°C oder -20°C bis 0°C zugegeben. Zur Vervollständigung der Umsetzung kann die Reaktionsmischung in der Folge auf eine höhere Temperatur, z.B. 45 bis 100°C oder 60 bis 90°C erhitzt werden.

Wenn es sich bei dem erfindungsgemäßen Verfahren um ein Verfahren zur Aufreinigung von ionischen Flüssigkeiten handelt, ist die Säure, die in Schritt iii) verwendet wird, die zum Anion der ionischen Flüssigkeit, die in Schritt i) eingesetzt wurde, korrespondierende Säure. In diesem Fall ist $X^-$ in $Im^+X^-$ und Y-in $Im^+Y^-$ gleich.

In einer Ausführungsform ist das erfindungsgemäße Verfahren ein Verfahren zur Aufreinigung von ionischen Flüssigkeiten (auch als "Recyclingverfahren" bezeichnet) mit $Im^+X^- = Im^+Y^-$. In einer anderen Ausführungsform ist das erfindungsgemäße Verfahren ein Verfahren zur Herstellung von ionischen Flüssigkeiten mit $Im^+X^- \neq Im^+Y^-$.

Im Schritt iv) werden im erfindungsgemäßen Verfahren gebildeter Alkohol und überschüssiges Salz abgetrennt, die bei der Umsetzung der Säure HY mit dem Addukts des Kations der ionischen Flüssigkeit und des Alkoholatanions, sowie überschüssigem Alkoholat generiert werden. Zusätzlich kann in diesem Schritt durch Steuerung der Polarität einer Mischung von erzeugter ionischer Flüssigkeit und Lösungsmittel auch ein Großteil von noch vorhandenen Verunreinigungen abgetrennt werden.

Für die Abtrennung von festen Salzen und Verunreinigungen, die als Festsubstanzen aus entsprechenden Mischungen ausfallen, kann auf die vorstehenden Ausführungen in Zusammenhang mit der Trennung von Salzen in Schritt ii) verwiesen werden. Auch im Schritt iv) kann es dabei zweckmäßig sein, abtrennte feste Substanzen nachzuwaschen, um die Ausbeute an gewünschter ionischer Flüssigkeit zu erhöhen.

Alkohol verbleibt bei einer solchen Trennung zusammen mit der im Verfahren erzeugten ionischen Flüssigkeit in Lösung und kann in der Folge destillativ aus dem Gemisch entfernt werden kann. "Destillativ" schließt hier auch Fälle einer Behandlung unter reduziertem Druck ein, bei der das Lösungsmittel verdampft und anschließend durch entsprechende Kühlung wieder kondensiert wird.

Alternativ kann der Alkohol auch vor der Abtrennung von festen Salzen und Verunreinigungen abgetrennt werden, wobei die gleichen Verfahren verwendet werden können. Eine vorherige Abtrennung hat dabei den Vorteil einer Reduzierung der polaren Komponenten in der Mischung auf die ionische Flüssigkeit, was die Abscheidung von Salzen und Verunreinigungen infolge einer Zugabe eines organischen Lösungsmittels unterstützen kann.

In einer ganz besonders bevorzugten Ausgestaltung des erfindungsgemäßen Verfahrens wird Schritt iv) in der Weise durchgeführt, dass ein leichtflüchtiges unpolares Lösungsmittel mit einer Siedetemperatur von weniger als 120°C zugesetzt wird, das zweckmäßig eine geringere Polarität aufweist als das in Schritt i) verwendetet organische Lösungsmittel, und aus dem Gemisch ausfallendes Salz und andere feste Substanzen durch Filtrieren aus der Reaktionsmischung entfernt wird. In diesem Fall kann die ionische Flüssigkeit nachfolgend durch Verdampfen des zugesetzten leichtflüchtigen unpolaren Lösungsmittels erhalten werden.

Alternativ ist es möglich, die Abtrennung der ionischen Flüssigkeit in Schritt iv) durch Extrahieren der Mischung mit einem apolaren organischen Lösungsmittel zu realisieren. Bei einer solchen Extraktion bleibt überschüssiges Salz aus Alkalimetall und $Y^-$ als Feststoff zurück. Bei dem hier oder in der vorstehenden Alternative verwendeten organischen bzw. apolaren organischen Lösungsmittel handelt es sich bevorzugt um ein Lösungsmittel, das aus der Gruppe umfassend Ether, Carbonsäureester, Ketone oder Nitrile ausgewählt ist. Ganz besonders bevorzugt als Lösungsmittel sind Carbonsäureester wie insbesondere Ethylacetat und Ethylformiat oder Ketone wie insbesondere Aceton. Die Siedetemperatur der verwendeten organischen Lösungsmittel liegt bevorzugt im Bereich von 50 bis 100°C und insbesondere 55 bis 80°C.

In einer weiteren besonders bevorzugten Ausgestaltung wird das in iv) abgetrennte Salz durch Zugabe eine Säure mit niedrigerem pKs Wert in die Säure HY zurückgebildet wird. Das in Schritt ii) abgetrennte Salz kann ebenfalls entsprechend in die Säure HY zurückgebildet werden, wenn das Verfahren als Verfahren zur Aufreinigung einer ionischen Flüssigkeit ausgestaltet ist.

Im Vorstehenden wurde bereits erwähnt, dass das erfindungsgemäße Verfahren mit besonderem Vorteil im Kontext eines Verfahrens eingesetzt werden kann, bei dem Cellulose bei erhöhter Temperatur in einer ionischen Flüssigkeit gelöst und durch Nassspinnen zu Cellulose-Filamenten verarbeitet wird. In einem solchen Verfahren wird die ionische Flüssigkeit nach dem Spinnvorgang in einem Koagulationsbad behandelt, wobei die ionische Flüssigkeit in die Flüssigkeit des Koagulationsbads übergeht und aus dieser durch Entfernen des Koagulationslösungsmittel (in der Regel Wasser) zurückgewonnen werden kann. Demzufolge betrifft die vorliegende Erfindung in einem weiteren Aspekt die Verwendung des vorstehend angegebenen Verfahrens zu Aufreinigung und Rückgewinnung von ionischen Flüssigkeiten, die in solchen Nassspinnverfahren zur Herstellung von Cellulosefilamenten (= Celluloseendlosfasern) verwendet wurden.

In einem noch weiteren Aspekt betrifft die vorliegende Erfindung ein Verfahren mit mindestens den folgenden Schritten:

i) Herstellen einer Lösung von Cellulose in einer ionischen Flüssigkeit $Im^+X^-$;

ii) Verspinnen der in i) hergestellten Lösung, wobei die Lösung durch eine Spinndüse in ein Koagulationsbad gefördert wird, das ein Koagulationslösungsmittel, insbesondere in Form von Wasser, enthält;

iii) Bilden von Cellulosefilamenten im Koagulationsbad, wobei die ionische Flüssigkeit in das Koagulationslösungsmittel aufgenommen wird,

iv) Rückgewinnung der ionischen Flüssigkeit aus der im Koagulationsbad gebildeten Mischung durch Abdestillieren des Koagulationslösungsmittels,

v) gegebenenfalls ein oder mehrfaches Wiederholen der Schritte i) bis iv) unter Verwendung der in Schritt iv) erhaltenen ionischen Flüssigkeit,

vi) Einbezug der erhaltenen ionischen Flüssigkeit, die infolge ihrer Verwendung in den Schritten i) bis iv) Verunreinigungen enthält, in ein Verfahren, wie es im vorstehenden angegeben wurde, d.h. ein Verfahren, bei dem die verunreinigte ionische Flüssigkeit wie folgt behandelt wird:

vii) Umsetzen einer Mischung, die eine ionische Flüssigkeit $Im^+X^-$ und eine oder mehrere Verunreinigungen enthält, mit einem stöchiometrischen Überschuss an Alkali- oder Erdalkalimetallalkoholat, das in einem organischen Lösungsmittel gelöst ist,

viii) Abtrennen des bei der Umsetzung erzeugten Salzes aus Alkali- oder Erdalkalimetallkation und $X^-$ aus der Reaktionsmischung,

ix) Umsetzen des resultierenden Produkts mit einer zur eingesetzten Alkali- oder Erdalkalimetallalkoholat äquimolaren Menge an Säure HY, und

x) Abtrennen von hierbei generiertem Alkohol und überschüssigem Salz aus Alkalimetall und $Y^-$ aus der Reaktionsmischung.

Für die Schritte vii) bis x) gelten die vorstehenden für das erfindungsgemäße Verfahren gemachten Angaben zu bevorzugten Ausgestaltungen und Ausführungsformen analog.

Nachfolgend wird die vorliegende Erfindung anhand von Ausführungsbeispielen näher illustriert, die jedoch nicht in irgendeiner Weise als limitierend oder beschränkend für den Schutzumfang der Anmeldung anzusehen sind.

Beispiel 1: Umsetzung EMIMCl in EMIMOCT unter Abtrennung von Natriumchlorid

Unter Rühren werden 27,8 g (0.19 mol) EMIMCl in 50 bis 100 ml Methanol mit 36.0 g (0.20 mol) Natriummethylat (30%ig, 5% Überschuss auf die Menge des EMIMCl) in Methanol versetzt. Nach dem Abkühlen auf 10 °C wird das gebildete Kochsalz (NaCl) abgesaugt und der Filterkuchen mit je 5-10 ml Methanol dreimal gewaschen und getrocknet.

Die vereinigten Filtrate werden unter Rühren mit ca. 28.8 g (0.20 mol) Octansäure versetzt, wobei sich der Ansatz erwärmt. Aus der Mischung wird im Rotationsverdampfer bei Normaldruck Methanol abdestilliert. Methanolreste werden im Vakuum bei 50 °C entfernt. Der entstandene gallertartige Rückstand wird zur Extraktion der ionischen Flüssigkeit mit 100 ml Methylformiat als Lösungsmittel versetzt, mit dem Spatel zerkleinert, kräftig geschüttelt und 0.5 h bei 40 °C gerührt. Nach 4-14 h Stehenlassen wird die puddingartige Masse abgesaugt. Der Filterrückstand wird dreimal mit je 10 ml des betreffenden Lösungsmittels gewaschen und getrocknet.

Die vereinigten Filtrate werden eingedampft (Rotationsverdampfer) und Reste des Lösungsmittels werden im Vakuum (bis 90 °C) entfernt (2-3 h). Als Rückstand wurde reines EMIMOct in einer Ausbeute von 92-99% mit $n_D^{20}$ zwischen 1.4800-1.4809 (Handelsprodukte Fa. proionic: $n_D^{20} = 1.4805$ ) erhalten.

Bei analogen Untersuchungen, bei denen Ethylformiat, Ethylacetat, Ethylmethylketon, Aceton, oder Acetonitril für die Extraktion des EMIMOct verwendet wurden, wurden vergleichbare Ausbeuten und Reinheiten erhalten.

**Patentansprüche**

1. Verfahren zur Aufreinigung oder Herstellung von ionischen Flüssigkeiten des Typs $Im^+Y^-$, **gekennzeichnet durch** die folgenden Schritte:

   vi) Umsetzen einer Mischung, die eine ionische Flüssigkeit $Im^+X^-$ und eine oder mehrere Verunreinigungen enthält, mit einem stöchiometrischen Überschuss an Alkali- oder Erdalkalimetallalkoholat, das in einem organischen Lösungsmittel gelöst ist,
   vii) Abtrennen des bei der Umsetzung erzeugten Salzes aus Alkali- oder Erdalkalimetallkation und $X^-$ aus der Reaktionsmischung,
   viii) Umsetzen des resultierenden Produkts mit einer zur eingesetzten Alkali- oder Erdalkalimetallalkoholat äquimolaren Menge an Säure HY, und
   ix) Abtrennen von hierbei generiertem Alkohol und überschüssigem Salz aus Alkalimetall und $Y^-$ aus der Reaktionsmischung.

2. Verfahren nach Anspruch 1, wobei der stöchiometrische Überschuss in Schritt i) dadurch erzeugt wird, dass die umzusetzende ionische Flüssigkeit als rein angenommen wird, und die Molmenge der ionischen Flüssigkeit durch Teilen der umzusetzenden Gewichtsmenge an verunreinigter ionischer Flüssigkeit durch das Molekulargewicht der ionischen Flüssigkeit ermittelt wird.

3. Verfahren gemäß Anspruch 1 oder 2, wobei die in Schritt i) eingesetzte Mischung als Verunreinigung eine oder mehrere Stoffe ausgewählt aus der Gruppe umfassend Alkalimetallsalze, Mono- und/oder Oligosaccharide, insbesondere in Form von oligomeren Glucosen, Proteinen und Schwebstoffe enthält.

4. Verfahren nach mindestens einem der Ansprüche 1 bis 3, wobei die in i) verwendete Mischung einen Anteil an Verunreinigungen im Bereich von 2 bis 40 Gew.-% und bevorzugt 5 bis 30 Gew.-% enthält.

5. Verfahren nach mindestens einem der vorangehenden Ansprüche, wobei das Mengenverhältnis nach Gewicht des organischen Lösungsmittels zu der ionischen Flüssigkeit inklusive Verunreinigungen in Schritt i) im Bereich von 5:1 bis 1:1, bevorzugt 4:1 bis 1,5:1 eingestellt wird.

**6.** Verfahren nach mindestens einem der vorangehenden Ansprüche, wobei das organische Lösungsmittel in Schritt i) mit der ionischen Flüssigkeit homogen mischbar ist, und bevorzugt ausgewählt ist aus der Gruppe umfassend Methanol, Ethanol, Methylacetat, Ethylacetat und Acetonitril.

**7.** Verfahren nach mindestens einem der vorangehenden Ansprüche, wobei das Abtrennen von Salz aus der Reaktionsmischung in Schritt ii) und/oder iv) durch Filtrieren und/oder Zentrifugieren erfolgt.

**8.** Verfahren nach mindestens einem der vorangehenden Ansprüche, wobei das Anion $X^-$ der ionischen Flüssigkeit ausgewählt ist aus der Gruppe umfassend $F^-$, $Cl^-$, $Br^-$, $ClO_3^-$, $ClO_4^-$, $CN^-$, $SCN^-$, $NO_2^-$, $NO_3^-$, $HCO_3^-$, $CO_3^{2-}$ $HSO_4^-$, $SO_4^{2-}$, $S_2O_3^{2-}$, $S_2O_4^{2-}$, $S_2O_5^{2-}$, $S_2O_6^{2-}$, $HPO_2^-$, $H_2PO_4^-$, $HPO_4^{-2-}$, $RN\text{-}CS_2^-$, $BF_4^-$, $SbCl_6^-$, $AlCl_4^-$, $FeCl_4^-$,

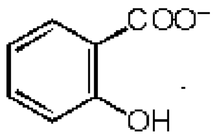

,

$C_nH_{2n+1}CO_2^-$, $C_nH_{2n-1}CO_2^-$, $C_nH_{2n-3}CO_2^-$, $^-O_2C\text{-}(CH_2)_n\text{-}CO_2H$, $^-O_2C\text{-}(CH_2)_n\text{-}CO_2^-$ mit n = 1 bis 25, $R\text{-}SO_3^-$, $CF_3SO_3^-$, $C_6H_5SO_3^-$, $C_nH_{2n-1}CO_2^-$,

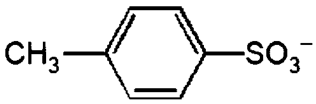

,

und $Ar\text{-}CO_2^-$ (Ar = $C_6H_5$,

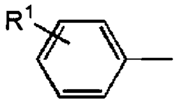

,

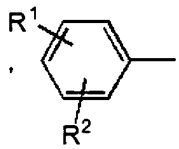

).

**9.** Verfahren nach mindestens einem der vorangehenden Ansprüche, **dadurch gekennzeichnet, dass** die Anionen der ionischen Flüssigkeiten des Typs $Im^+Y^-$ und $Im^+Y^-$ gleich sind, und das Verfahren als Verfahren zur Aufreinigung der ionischen Flüssigkeit durchgeführt wird.

**10.** Verfahren nach mindestens einem der vorangehenden Ansprüche, **dadurch gekennzeichnet, dass** es sich bei der in Schritt iii) eingesetzten Säure um eine von $F^-$, $Cl^-$, $Br^-$, $ClO_3^-$, $ClO_4^-$, $CN^-$, $SCN^-$, $NO_2^-$, $NO_3^-$, $HCO_3^-$, $CO_3^{2-}$, $HSO_4^-$ , $SO_4^{2-}$, $S_2O_3^{2-}$, $S_2O_4^{2-}$, $S_2O_5^{2-}$ , $S_2O_6^{2-}$, $HPO_2^-$, $H_2PO_4^-$, $HPO_4^{-2-}$, $RN\text{-}CS_2^-$, $BF_4^-$, $SbCl_6^-$, $AlCl_4^-$, $FeCl_4^-$,

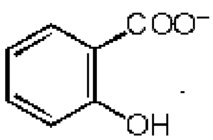

,

$C_nH_{2n+1}CO_2^-$, $C_nH_{2n-1}CO_2^-$, $C_nH_{2n-3}CO_2^-$, $^-O_2C\text{-}(CH_2)_n\text{-}CO_2H$, $^-O_2C\text{-}(CH_2)_n\text{-}CO_2^-$ mit n = 1 bis 25, $R\text{-}SO_3^-$, $CF_3SO_3^-$, $C_6H_5SO_3^-$, $C_nH_{2n-1}CO_2^-$,

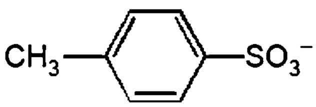

,

und $Ar\text{-}CO_2^-$ (Ar = $C_6H_5$,

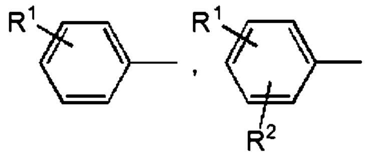

) abgeleiteten Protonensäure handelt.

**11.** Verfahren nach Anspruch 10, **dadurch gekennzeichnet, dass** es sich bei der in Schritt iii) eingesetzten Säure um eine Carbonsäure, bevorzugt um eine gesättigte oder ungesättigte C4 bis C20 Carbonsäure und bevorzugt um eine gesättigte oder ungesättigte C6 bis C18 Carbonsäure handelt.

**12.** Verfahren nach mindestens einem der vorangehenden Ansprüche, wobei für das Abtrennen von generiertem überschüssigem Salz aus Alkali- oder Erdalkalimetall und $Y^-$ in Schritt iv) ein unpolares Lösungsmittel mit einer Siedetemperatur von weniger als 120°C zugesetzt wird, das bevorzugt eine geringere Polarität aufweist als das in Schritt i) verwendete organische Lösungsmittel, und aus dem Gemisch ausfallendes Salz durch Filtrieren aus der Reaktionsmischung entfernt wird, wobei bevorzugt die ionische Flüssigkeit nachfolgend durch Verdampfen des zugesetzten leichtflüchtigen unpolaren Lösungsmittels generiert wird.

**13.** Verfahren nach mindestens einem der vorangehenden Ansprüche, **dadurch gekennzeichnet, dass** die Abtrennung der ionischen Flüssigkeit in Schritt iv) durch Extrahieren mit einem apolaren organischen Lösungsmittel, bevorzugt in Form von Ethylacetat oder Aceton, erfolgt, wobei überschüssiges Salz aus Alkali- oder Erdalkalimetall und $Y^-$ als Feststoff zurückbleibt.

**14.** Verfahren nach mindestens einem der vorangehenden Ansprüche, wobei das in Schritt ii) und/oder iv) abgetrennte Salz durch Zugabe eine Säure mit niedrigerem pKs Wert in die Säure HY zurückgebildet wird.

**15.** Verwendung eines Verfahrens gemäß einem der Ansprüche 1 bis 14 zur Aufreinigung und Rückgewinnung von ionischen Flüssigkeiten, die in Nassspinnverfahren zur Herstellung von Cellulosefilamenten verwendet wurden.

Europäisches Patentamt
European Patent Office
Office européen des brevets

# EUROPÄISCHER RECHERCHENBERICHT

Nummer der Anmeldung
EP 24 22 1208

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D<br>Y | WO 2024/153790 A1 (TECHNIKUM LAUBHOLZ GMBH [DE]) 25. Juli 2024 (2024-07-25)<br>* Seite 1; Ansprüche 1-3; Beispiel 2 * | 1-14<br>1-15 | INV. C07D233/58 |
| Y | CN 102 516 177 A (NANJING UNIVERSITY OF TECHNOLOGY ET AL.) 27. Juni 2012 (2012-06-27)<br>* Tabelle 1 * | 1-14 | |
| X,D | WO 2005/070896 A1 (BASF AG [DE]; MAASE MATTHIAS [DE] ET AL.) 4. August 2005 (2005-08-04)<br>* Seite 1; Anspruch 1; Beispiel 1 * | 1-14 | |
| Y | GB 2 502 607 A (UNIV LEUVEN KATH [BE]) 4. Dezember 2013 (2013-12-04)<br>* letzter Absatz;<br>Seite 3089 * | 1-14 | |
| Y | AZIMI BAHAREH ET AL: "Cellulose-based fiber spinning processes using ionic liquids", CELLULOSE, SPRINGER NETHERLANDS, NETHERLANDS, Bd. 29, Nr. 6, 7. März 2022 (2022-03-07), Seiten 3079-3129, XP037808726, ISSN: 0969-0239, DOI: 10.1007/S10570-022-04473-1 [gefunden am 2022-03-07]<br>* Seite 3089 - Seite 3090 * | 15 | RECHERCHIERTE SACHGEBIETE (IPC)<br>C07D |

Der vorliegende Recherchenbericht wurde für alle Patentansprüche erstellt

2

| Recherchenort | Abschlußdatum der Recherche | Prüfer |
|---|---|---|
| München | 5. Juni 2025 | Grassi, Damian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 24 22 1208

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-06-2025

| Im Recherchenbericht angeführtes Patentdokument | Datum der Veröffentlichung | Mitglied(er) der Patentfamilie | Datum der Veröffentlichung |
|---|---|---|---|
| WO 2024153790 A1 | 25-07-2024 | DE 102023101455 A1 | 25-07-2024 |
| | | WO 2024153790 A1 | 25-07-2024 |
| CN 102516177 A | 27-06-2012 | KEINE | |
| WO 2005070896 A1 | 04-08-2005 | AT E510826 T1 | 15-06-2011 |
| | | CN 1914181 A | 14-02-2007 |
| | | DE 102004003958 A1 | 11-08-2005 |
| | | EP 1711472 A1 | 18-10-2006 |
| | | ES 2365347 T3 | 30-09-2011 |
| | | JP 4980726 B2 | 18-07-2012 |
| | | JP 2007518772 A | 12-07-2007 |
| | | KR 20060128948 A | 14-12-2006 |
| | | US 2007142646 A1 | 21-06-2007 |
| | | US 2011065926 A1 | 17-03-2011 |
| | | WO 2005070896 A1 | 04-08-2005 |
| GB 2502607 A | 04-12-2013 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005070896 A1
- WO 2024153790 A1
- WO 2005070896 A


### In der Beschreibung aufgeführte Nicht-Patentliteratur


- **K. R. SEDDON**. Ionic Liquids for Clean Technology. *J. Chem. Tech. Biotechnol.*, 1997, vol. 68, 351-356
- **N. N. ENE**. Room Temperature Molten Salts. *Roum. Chem. Q. Rev.*, 1993, vol. 1, 333-351
- **P. TEISEN et al.** *Electrochemical Society Proceedings*, vol. 99-41, 161-168